# EUROPEAN PATENT APPLICATION

(11) **EP 4 360 480 A1**
(43) Date of publication of application: **01.05.2024**
(21) Application number: 21946990.5
(22) Date of filing: 21.06.2021
(51) Int. Cl.: A24F 40/42, A24F 40/50

(54) **CONTROLLER FOR SUCTION APPARATUS**

(71) Applicant: Japan Tobacco, Inc., Tokyo, 105-6927 (JP)
(72) Inventor: FUJINAGA, Ikuo, Tokyo 130-8603 (JP); YAMADA, Kentaro, Tokyo 130-8603 (JP); FUJITA, Hajime, Tokyo 130-8603 (JP)
(74) Representative: Hoffmann Eitle
(86) International application number: PCT/JP2021/023446
(87) International publication number: WO 2022/269700

(57) **Abstract**

A controller for an inhalation apparatus, which operates by electric power supplied from a power supply, includes a holding portion configured to hold an atomizer including a heater configured to generate flavored aerosol from a generation source of an aerosol source, a nonvolatile display; and a processor configured to control update of display of the nonvolatile display, wherein the processor determines whether to perform the update in response to occurrence of a factor for changing a remaining amount of the generation source.

## Description

### TECHNICAL FIELD

The present invention relates to a controller for an inhalation apparatus.

### BACKGROUND ART

In an aerosol generation apparatus that generates aerosol that can be inhaled, the number of puff operations (inhalation operations) that can be performed by one charge can be one important indicator. To improve such indicator, it is important to reduce the power consumption in a display unit and the like provided in the aerosol generation apparatus.

PTL 1 describes that electronic paper (e-ink) is adopted for the display unit of an electronic cigarette to reduce the power consumption. In addition, PTLs 2 and 3 describe that e-ink can be adopted for the display unit of an inhalation apparatus. However, PTLs 1, 2, and 3 do not disclose the timing or condition of updating display of electronic paper, and thus provide no technical meaning other than the use of electronic paper as a display device.

### CITATION LIST

### PATENT LITERATURE

PTL 1: Chinese Utility Model Registration No. 203505584
PTL 2: US-2017-0304567
PTL 3: U.S. Patent No. 8851068

### SUMMARY OF INVENTION

### TECHNICAL PROBLEM

One aspect of the present invention provides a preferable example of control of a nonvolatile display in a controller for an inhalation apparatus.

One aspect of the present invention is related to a controller for an inhalation apparatus, which operates by electric power supplied from a power supply, and the controller for the inhalation apparatus comprises: a holding portion configured to hold an atomizer including a heater configured to generate flavored aerosol from a generation source of an aerosol source; a nonvolatile display; and a processor configured to control update of display of the nonvolatile display, wherein the processor determines whether to perform the update in response to occurrence of a factor for changing a remaining amount of the generation source.

In one embodiment, the factor includes discharge from the power supply to the heater, and the processor performs, after an end of the discharge, the update of the display of the nonvolatile display while the discharge is not performed.

In one embodiment, the processor does not perform the update of the display of the nonvolatile display while the discharge is performed.

In one embodiment, the display of the nonvolatile display includes display concerning a remaining amount of the power supply.

In one embodiment, the factor includes replacement of the generation source.

In one embodiment, the display of the nonvolatile display includes display concerning the remaining amount of the generation source.

In one embodiment, the generation source includes a first generation source as a generation source of aerosol, and a second generation source as a generation source of a flavor, the factor includes replacement of the first generation source, and the factor includes replacement of the second generation source.

In one embodiment, the display of the nonvolatile display includes display concerning a remaining amount of the first generation source and display concerning a remaining amount of the second generation source.

In one embodiment, the display of the nonvolatile display includes display concerning a remaining amount of at least one element consumed to generate the flavored aerosol.

In one embodiment, the display of the nonvolatile display includes bar graph display.

In one embodiment, the display of the nonvolatile display includes display for specifying one of two states with respect to at least one of the at least one element.

In one embodiment, the two states include a first state indicating that a capability of generating the flavored aerosol is sufficient and a second state indicating that the capability of generating the flavored aerosol is insufficient.

In one embodiment, the processor includes a first mode in which discharge from the power supply to the heater can be controlled and a second mode in which power consumption is smaller than in the first mode, the display of the nonvolatile display includes generation source remaining amount display concerning the remaining amount of the generation source, and in a case where the generation source is replaced in the second mode, the processor updates the generation source remaining amount display.

In one embodiment, in a case where the generation source is replaced in the second mode, the processor updates the generation source remaining amount display after shifting to the first mode.

In one embodiment, the processor detects, as replacement of the generation source, that the generation source is detached from the holding portion and then a new generation source is attached to the holding portion.

In one embodiment, the processor detects replacement of the generation source based on an electrical signal obtained from a current path formed by holding the generation source by the holding portion.

In one embodiment, the controller for the inhalation apparatus, further comprises an operation unit, wherein the processor shifts from the second mode to the first mode in response to an operation of the operation unit, and the processor includes an input terminal configured to be supplied with the electrical signal obtained from the current path and a signal corresponding to an output signal of the operation unit, and detects a command of shifting from the second mode to the first mode and replacement of the generation source based on the signals supplied to the input terminal.

In one embodiment, the controller for the inhalation apparatus, further comprises a sensor configured to detect presence or absence of the generation source, wherein the processor detects replacement of the generation source based on an output from the sensor.

In one embodiment, the controller for the inhalation apparatus, further comprises an operation unit, wherein the processor shifts from the second mode to the first mode in response to an operation of the operation unit, and in a case where the generation source is replaced in the second mode, the processor shifts to the first mode in response to an operation of the operation unit, and then updates the generation source remaining amount display.

In one embodiment, the controller for the inhalation apparatus, the processor includes a first mode in which discharge from the power supply to the heater can be controlled and a second mode in which power consumption is smaller than in the first mode, the display of the nonvolatile display includes generation source remaining amount display concerning the remaining amount of the generation source, and the processor shifts from the second mode to the first mode at a scheduled timing to confirm replacement of the generation source, and updates, in a case where the generation source is replaced, the generation source remaining amount display.

In one embodiment, a frequency of performing the update by the processor is lower than a frequency of discharge from the power supply to the heater.

### BRIEF DESCRIPTION OF DRAWINGS

Fig. 1 is an exploded perspective view of an inhalation apparatus;
Fig. 2 is an assembly completed diagram of the inhalation apparatus;
Fig. 3 is a view of the internal arrangement of the inhalation apparatus;
Fig. 4 is a circuit diagram showing an example of the arrangement of an electrical component incorporated in the inhalation apparatus;
Fig. 5 shows a state transition diagram of the inhalation apparatus or a power supply unit, and views of display examples of a display;
Fig. 6 is a view showing display examples of the display;
Fig. 7 is a flowchart illustrating an example of the operation of the power supply unit;
Fig. 8 is a flowchart illustrating the example of the operation of the power supply unit;
Fig. 9 is a flowchart illustrating the example of the operation of the power supply unit;
Fig. 10 is a flowchart illustrating the example of the operation of the power supply unit;
Fig. 11 is a flowchart illustrating the example of the operation of the power supply unit;
Fig. 12 is a flowchart illustrating the example of the operation of the power supply unit;
Fig. 13 is a flowchart illustrating the example of the operation of the power supply unit;
Fig. 14 is a circuit diagram showing another example of the arrangement of the electrical component incorporated in the inhalation apparatus;
Fig. 15 is a circuit diagram for explaining the operation of the other example of the arrangement of the electrical component incorporated in the inhalation apparatus;
Fig. 16 is a circuit diagram for explaining the operation of the other example of the arrangement of the electrical component incorporated in the inhalation apparatus; and
Fig. 17 is a flowchart illustrating an example of the operation in the example of the arrangement shown in Fig. 14.

### DESCRIPTION OF EMBODIMENTS

Hereinafter, embodiments will be described in detail with reference to the attached drawings. Note, the following embodiments are not intended to limit the scope of the claimed invention, and limitation is not made to an invention that requires a combination of all features described in the embodiments. Two or more of the multiple features described in the embodiments may be combined as appropriate. Furthermore, the same reference numerals are given to the same or similar configurations, and redundant description thereof is omitted.

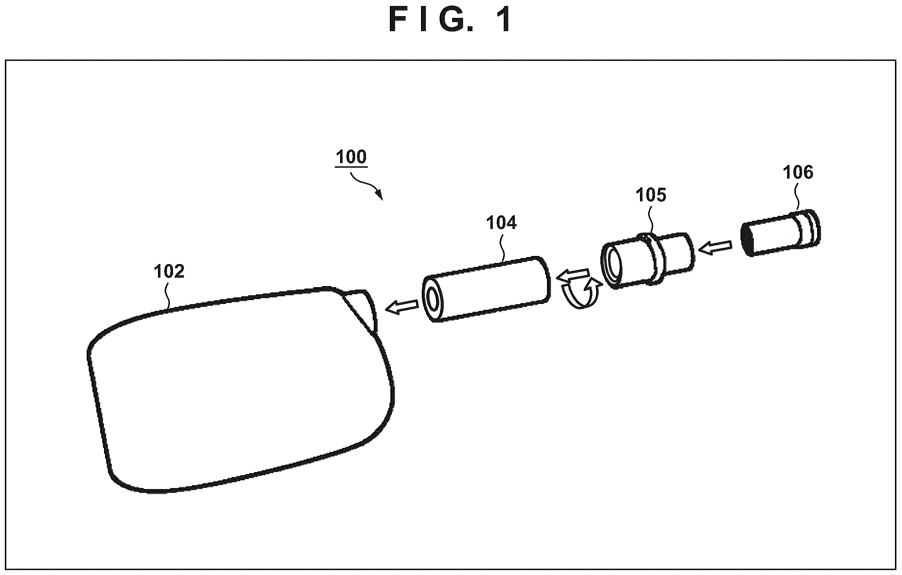

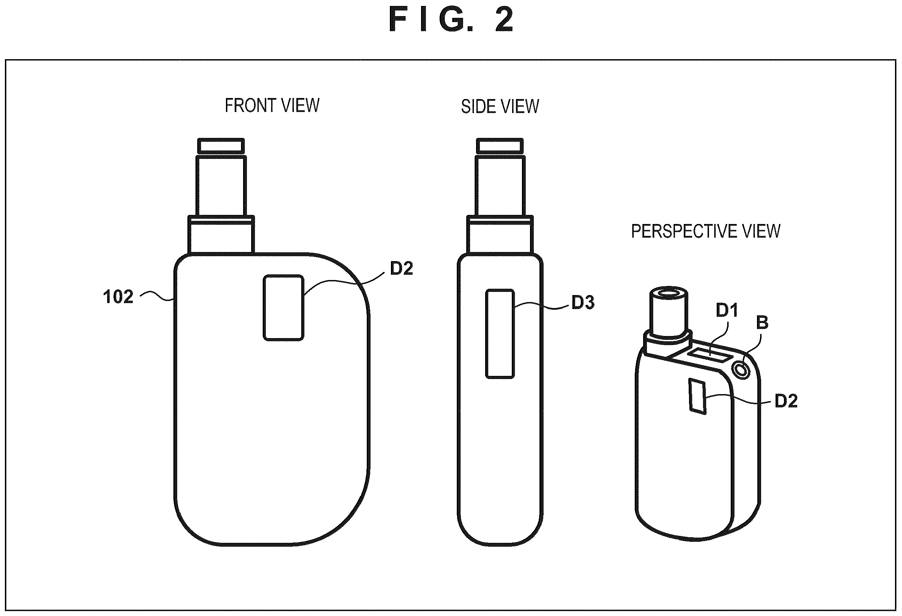

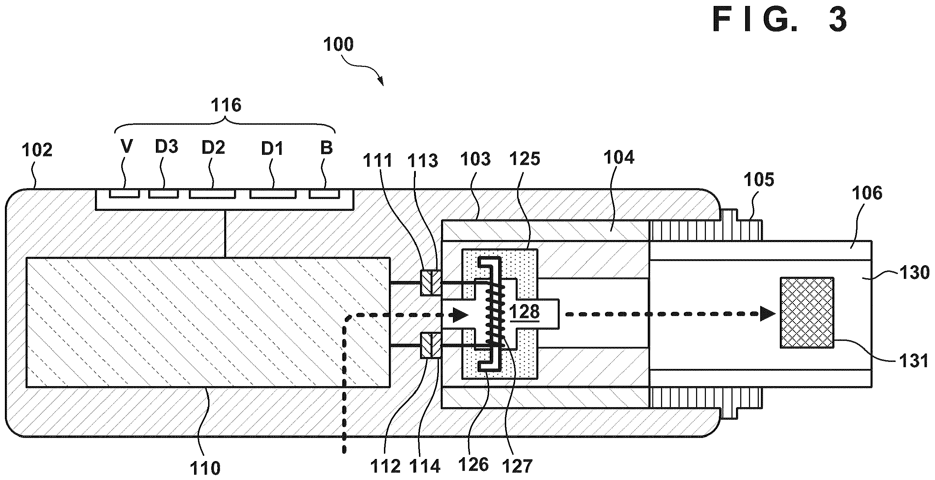

The arrangement of a inhalation apparatus 100 as one form of an aerosol generation apparatus will be described with reference to Figs. 1, 2, and 3. Fig. 1 shows an exploded perspective view of the inhalation apparatus 100, Fig. 2 shows an assembly completed diagram (front view, side view, and perspective view) of the inhalation apparatus 100, and Fig. 3 shows a view of the internal arrangement of the inhalation apparatus 100. The inhalation apparatus 100 can be configured to provide, to a user via a mouthpiece portion 130, aerosol, flavored aerosol, a gas containing aerosol and a flavor material, aerosol, or aerosol containing a flavor material in accordance with an operation of requesting generation of aerosol (to be also referred to as an "atomization request" hereinafter) such as a suction operation by the user. The inhalation apparatus 100 can include a power supply unit 102 as a controller for the inhalation apparatus, an atomizer 104, a capsule holder 105, and a capsule 106.

The atomizer 104 can be configured to generate flavored aerosol from the generation source of an aerosol source. The aerosol source can be, for example, a liquid such as a polyhydric alcohol such as glycerin or propylene glycerol. Alternatively, the aerosol source may contain a drug. The aerosol source may be a liquid, a solid, or a mixture of a liquid and a solid. A vapor source such as water may be used in place of the aerosol source. The atomizer 104 may be provided as a cartridge detachable from the power supply unit 102. The atomizer 104 may be provided not to be detachable from the power supply unit 102. In this specification, the atomizer 104 will sometimes be referred to as the cartridge 104 hereinafter. The power supply unit 102 may be understood as a driving unit that drives the atomizer 104, a holding body that holds the atomizer 104, a main body that causes the atomizer 104 to function, or the like.

The power supply unit 102 can include a holding portion 103 that holds the atomizer 104. The holding portion 103 can be configured to accommodate the overall atomizer 104 or a part of it. The holding portion 103 may further be configured to hold the capsule holder 105. Alternatively, the capsule holder 105 may be held by the atomizer 104. The capsule holder 105 holds the capsule 106. The capsule holder 105 may function to fix the atomizer 104 in cooperation with the holding portion 103.

The holding portion 103 may include a lock mechanism for preventing the capsule holder 105 from dropping from the holding portion 103. The lock mechanism can include a second engagement portion configured to engage with a first engagement portion that can be provided in the capsule holder 105. In a state in which the lock mechanism operates, that is, a state in which the capsule holder 105 is locked by the lock mechanism, connecting portions 113 and 114 of the atomizer 104 are pressed against connecting portions 111 and 112 of the power supply unit 102, respectively, and electrical connections between the connecting portions 113 and 114 and the connecting portions 111 and 112 can thus be provided.

The capsule holder 105 may be integrated with the atomizer 104 or the power supply unit 102. Furthermore, if the capsule 106 is inserted into the opening portion of the capsule holder 105, a gas can circulate between the atomizer 104 and the capsule 106. The capsule 106 can include a flavor source 131. The flavor source 131 can be, for example, a molded body formed by molding a tobacco material. Alternatively, the flavor source 131 may be formed by a plant (for example, mint, herb, Chinese herb, coffee bean, and the like) other than tobacco. A flavor such as menthol may be added to the flavor source. The flavor source 131 may be added to the aerosol source. The user can hold, in the mouth, the mouthpiece portion 130 formed at the distal end of the capsule 106 and suck flavored aerosol.

The power supply unit 102 can include an electrical component 110. The electrical component 110 can include a user interface 116. Alternatively, the power supply unit 102 may be understood to include the electrical component 110 and the user interface 116. The user interface 116 can include an action button B as an operation unit operable by the user. The action button B can be a button used as a trigger for an operation such as activation of the power supply unit 102 or display of information.

The user interface 116 can further include a first display D1 as a first notification unit and a second display D2 as a second notification unit. The first display D1 and the second display D2 may have different display principles. In this case, the power consumption of the first display D1 is different from that of the second display D2. For example, the first display D 1 can be an Organic Light Emitting Diode (OLED) display, and the second display D1 can be an electronic paper (e-ink) display. The electronic paper (e-ink) display is a kind of nonvolatile display. The OLED display that can be adopted as the first display D1 does not require a backlight device that is required by a liquid crystal display since the organic light emitting diode emits light. The electronic paper display that can be adopted as the second display D2 does not require electric power to continuously hold an image. Therefore, the power consumption of the electronic paper display is smaller than that of the OLED display. However, the electronic paper display has no self-light emitting function unlike the OLED display, and thus has low visibility in a dark environment, as compared with the OLED display.

The user interface 116 can further include a third notification unit in addition to the first display D1 and the second display D2. The third notification unit can include a third display D3 and/or a vibration generation unit V As the third display D3, for example, a Light Emitting Diode (LED) display can be adopted. In an example, the LED display can be formed by 10 or less, 20 or less, or 30 or less LEDs. In this case, the LED display can display a small amount of information, as compared with the electronic paper display, but has high visibility since high luminance can be implemented. Alternatively, the LED display may be a display formed by an LED array.

The vibration generation unit V can be formed by a vibration motor for vibrating the housing of the power supply unit 102. By vibrating the housing by the vibration motor, it is possible to notify the user, who holds the housing, of the state of the power supply unit 102. In an example, the power consumption of the first display D1 and the power consumption of the third display are larger than that of the second display D2, and the power consumption of the first display D1 is larger than that of the third display D3 or the vibration generation unit V

Fig. 2 shows an example of the arrangement of the action button B, the first display D 1, the second display D2, and the third display D3. In the example shown in Fig. 2, the first display D1 is arranged on the upper surface of the power supply unit 102, and the second display D2 and the third display D3 are arranged on different side surfaces of the power supply unit 102. However, the arrangement positions of the displays D1, D2, and D3 may be changed, or the displays D1, D2, and D3 may be arranged at positions different from those shown in Fig. 2. The third display D3 (for example, an LED display) may be arranged around a window portion for viewing the remaining amount of the aerosol source in the atomizer 104, as shown in, for example, the side view of Fig. 2. Alternatively, the third display D3 may be arranged around the action button B. Furthermore, the arrangement position of the action button B is not limited to that shown in the example of Fig. 2, and the action button B may be arranged at another position.

The power supply unit 102 can include the first connecting portion 111 and the second connecting portion 112. In a state in which the atomizer 104 is held by the holding portion 103 and the capsule holder 105 is attached to the holding portion 103, the first connecting portion 111 can electrically be connected to the third connecting portion 113 of the atomizer 104, and the second connecting portion 112 can electrically be connected to the fourth connecting portion 114 of the atomizer 104. The first connecting portion 111, the second connecting portion 112, the third connecting portion 113, and the fourth connecting portion 114 can be electrical contacts or connectors. The power supply unit 102 can supply electric power to the atomizer 104 via the first connecting portion 111 and the second connecting portion 112.

The atomizer 104 can include the third connecting portion 113 and the fourth connecting portion 114. Furthermore, the atomizer 104 can include a heater 127 for generating flavored aerosol from the generation source of the aerosol source, a container 125 that holds the aerosol source, and a transport portion 126 that transports the aerosol source held by the container 125 to a heating area by the heater 127 and holds the aerosol source in the heating area. At least a part of the heating area can be arranged in a channel 128 provided in the atomizer 104. The first connecting portion 111, the third connecting portion 113, the heater 127, the fourth connecting portion 114, and the second connecting portion 112 form a current path configured to flow a current to the heater 127. The transport portion 126 can be made of, for example, a fiber material such as glass fiber, a porous material such as ceramic, or a combination thereof. Note that the transport portion 126 can also be called a wick. Note that the means for transporting the aerosol source in the container 125 to the heating area is not limited to the wick, and may be implemented by a spraying device such as a spray or a transporting means such as a pump.

If the user holds the mouthpiece portion 130 in the mouth and performs a suction operation, air flows into the channel 128 of the atomizer 104 through an opening (not shown), as exemplified by a broken arrow. When the heater 127 heats the aerosol source, the vaporized and/or aerosolized aerosol source is transported toward the mouthpiece portion 130 by air. In the process in which the aerosol source is transported toward the mouthpiece portion 130, the vaporized aerosol source is cooled to form fine liquid droplets, thereby promoting aerosolization. In the arrangement in which the flavor source 131 is arranged, a flavor material generated from the flavor source 131 is added to the aerosol, and the resultant flavored aerosol is transported to the mouthpiece portion 130, and inhaled into the user's mouth. Since the flavor material generated from the flavor source 131 is added to the aerosol, the flavor material can efficiently be transported to the lungs of the user without staying in the oral cavity of the user.

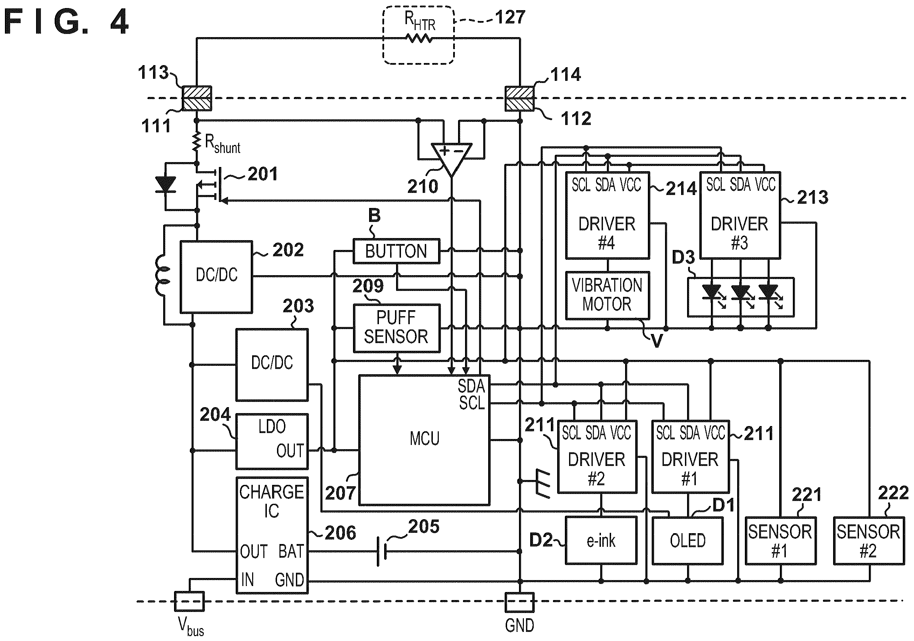

Fig. 4 shows an example of the arrangement of the electrical component 110. The electrical component 110 can include a power supply 205 and a charging circuit 206. The power supply 205 can be a chargeable battery (secondary battery) such as a lithium-ion secondary battery. Alternatively, the power supply 205 may be formed by an electric double-layer capacitor such as a lithium-ion capacitor. The power supply 205 can be charged using, for example, electric power supplied from a V_{bus} port. A power supply device (external power supply) (not shown) can be connected to the V_{bus} port via a cable. The V_{bus} port, the cable, and the power supply device can be formed in compliance with, for example, standards such as the Universal Serial Bus (USB) Type-A, Type-B, and Type-C. The power supply device can supply electric power to the power supply 205 via the cable and the V_{bus} port. The power supply device can be a personal computer (PC) or a charger such as a portable battery. When the power supply unit 102 is connected to the charger via the V_{bus} port and the cable, the power supply unit 102 and the charger communicate with each other, and then the charger can charge the power supply 205. Note that for connection between the power supply unit 102 and the power supply device, not only the USB interface but also other various communication methods that can implement data communication and electric power supply can be applied.

The charging circuit 206 can supply a charge current from a charging terminal BAT to the power supply 205 using electric power supplied from the power supply device to an input terminal IN, thereby charging the power supply 205. Furthermore, the charging circuit 206 can output electric power or a voltage from a voltage output terminal OUT using the electric power supplied from the power supply device. Therefore, even in charging of the power supply 205 by the charging circuit 206, the charging circuit 206 can supply the electric power or voltage to voltage converters 202, 203, and 204. In a case where the power supply device is not connected to the Vbus port, the charging circuit 206 can output the electric power or voltage from the voltage output terminal OUT using the electric power of the power supply 205 supplied from the power supply 205 to the charging terminal BAT. Thus, during a period other than a period in which the power supply 205 is charged by the charging circuit 206, the charging circuit 206 can supply the electric power or voltage to the voltage converters 202, 203, and 204.

The electrical component 110 can include one or a plurality of voltage converters. In a case where the electrical component 110 includes a plurality of voltage converters, at least two of the voltage converters can generate different voltages or equal voltages. In the example of the arrangement shown in Fig. 4, the electrical component 110 includes the voltage converters 202, 203, and 204. The electrical component 110 need not include any voltage converter. In this case, a voltage output from the power supply 205 can be provided to a plurality of elements forming the electrical component 110 with a small voltage drop caused by the wire resistance. In the example of the arrangement shown in Fig. 4, the voltage converters 202 and 203 are each formed by a switching regulator such as a DC/DC converter but at least one of the voltage converters may be formed by an LDO (Low DropOut) or a circuit of another system. In the example of the arrangement shown in Fig. 4, the voltage converter 204 is formed by an LDO (Low DropOut) but may be formed by a switching regulator such as a DC/DC converter or a circuit of another system.

The electrical component 110 can include a processor 207 that operates in accordance with software (program) installed in advance. The processor 207 can be configured to control the plurality of elements forming the electrical component 110. The processor 207 can be formed by, for example, an MCU (Micro Controller Unit). The processor 207 may be replaced by another device such as an ASIC. The processor 207 can be supplied with a voltage (electric power) from the voltage converter 204. The processor 207 can be configured to control drivers 211, 212, 213, and 214 as elements forming the electrical component 110. The drivers 211, 212, 213, and 214 drive the displays D1, D2, and D3 and the vibration motor V, respectively. Therefore, the processor 207 may be understood as a processor that drives or controls the displays D1, D2, and D3 and the vibration motor V

The processor 207 can be configured to determine whether to update the display of the second display D2 in accordance with the occurrence of a factor for changing the remaining amount of the generation source of aerosol. Alternatively, the processor 207 can be configured to determine whether to update the display of at least one of the first display D1 and the third display D3 in addition to the second display D2 in accordance with the occurrence of a factor for changing the remaining amount of the generation source of aerosol. The factor for changing the remaining amount of the generation source of aerosol can be, for example, heating of the aerosol source that is performed to generate aerosol, in other words, supply (discharge from the power supply 205 to the heater 127) of electric power to the heater 127 that is performed to generate aerosol. Alternatively, the factor for changing the remaining amount of the generation source of aerosol can include replacement of the cartridge 104 and replacement of the capsule 106.

Determining whether to update the display of the second display D2 in accordance with the occurrence of the factor for changing the remaining amount of the generation source of aerosol is advantageous in preventing determination of the update from being performed excessively frequently and suppressing the power consumption of the power supply unit 102. Furthermore, determining whether to update the display of the second display D2 in accordance with the occurrence of the factor for changing the remaining amount of the generation source of aerosol is advantageous in quickly updating the display of the second display D2 in response to the occurrence of the factor for changing the remaining amount of the generation source of aerosol. The frequency of updating the display of the second display D2 by the processor 207 in accordance with the occurrence of the factor for changing the remaining amount of the generation source of aerosol may be lower than the frequency of discharge from the power supply 205 to the heater 127.

The atomizer 104 may be understood as a first generation source as the generation source of aerosol. The capsule 106 may be understood as a second generation source of flavored aerosol since it adds a flavor to aerosol. Alternatively, the second generation source may be understood as the generation source of a flavor. The information providing function formed by the displays D1, D2, and D3 may be implemented by one or a plurality of nonvolatile displays such as electronic paper displays. Alternatively, the factor for changing the remaining amount of the generation source of aerosol may be detection of puff by a puff sensor 209 or an operation of responding to the detection. The processor 207 can operate to update the display of all or some (for example, the second display D2) of the displays D1, D2, and D3 while discharge from the power supply 205 to the heater 127 is not performed after the end of the discharge. On the other hand, the processor 207 can operate not to update the display of the displays D1, D2, and D3 while discharge from the power supply 205 to the heater 127 is performed.

The drivers 211, 212, 213, and 214 can be supplied with the voltage (electric power) from the voltage converter 204. The voltage converter 204 can also supply the voltage (electric power) to the action button B. When the action button B is set from the OFF state to the ON state by the user, it can supply a signal of an active level (for example, high level) to the processor 207. The first display D1 that can be formed by an OLED can be supplied with a voltage (electric power) from the voltage converter 203. In the example shown in Fig. 4, the displays D2 and D3 and the vibration motor V can be supplied with voltages (electric power) from the drivers 212, 213, and 214, respectively. Alternatively, at least one of the displays D2 and D3 and the vibration motor V may be supplied with the voltage (electric power) from one of the voltage converters 202, 203, and 204.

The power supply unit 102 may include the puff sensor 209 that detects a suction operation by the user, that is, puff, and the voltage converter 204 can supply the voltage (electric power) to the puff sensor 209. The puff sensor 209 can detect puff by detecting, for example, at least one of the pressure, the sound, and the temperature (for example, the temperature of air flowing into the channel 128 of the atomizer 104 via the above-described opening, or the temperature of the heater 127).

The electrical component 110 can include a switch 201 for controlling energization (supply of electric power) to the heater 127 as the load of the atomizer 104. The switch 201 can be, for example, a MOSFET including a body diode but may be formed by another switching element. The voltage converter 202 can supply a voltage (electric power) to the heater 127 via the switch 201. A shunt resistor Rₛₕᵤₙₜ may be arranged in a current path or a closed circuit formed by including the switch 201 and the heater 127.

The electrical component 110 can include a measurement circuit 210 for measuring the temperature of the heater 127. The heater 127 can have a positive or negative temperature coefficient characteristic in which a resistance value R_{HTR} changes in accordance with the temperature of the heater 127, and the resistance value R_{HTR} of the heater 127 can have a strong correlation with the temperature of the heater 127. The measurement circuit 210 is a circuit for measuring the resistance value R_{HTR} of the heater 127, and can be configured to, for example, measure the voltages at the two terminals of the heater 127 by an operational amplifier. The output from the measurement circuit 210 is provided to the processor 207, and the processor 207 can calculate the resistance value R_{HTR} based on the output from the measurement circuit 210 and a current value flowing through the heater 127. The current value can be obtained using, for example, a detection circuit that detects the voltages at the two terminals of the shunt resistor Rₛₕᵤₙₜ. The detection circuit may be, for example, a circuit that detects the potential difference between the two terminals of the shunt resistor Rₛₕᵤₙₜ, and can be provided as, for example, an AD converter in the processor 207. The processor 207 can control the switch 201 so as to perform feedback control, for example, PID control of the temperature of the heater 127 based on the temperature of the heater 127 measured using the measurement circuit 210.

The electrical component 110 may include a first sensor 221 that detects the presence or absence of the cartridge 104 and a second sensor 222 that detects the presence or absence of the capsule 106. Outputs from the first sensor 221 and the second sensor 222 can be provided to the processor 207. The first sensor 221 and the second sensor 222 can be supplied with the voltage (electric power) from the voltage converter 204. Each of the first sensor 221 and the second sensor 222 can be, for example, a photo interrupter, a proximity sensor, an RFID system, or a switch. The switch that detects the presence or absence of the cartridge 104 can be turned on (or off) when the cartridge 104 is inserted into the holding portion 103, and turned off (or on) when the atomizer 104 is detached from the holding portion 103. The switch that detects the presence or absence of the capsule 106 can be turned on (or off) when the capsule 106 is inserted into the holding portion 103, and turned off (or on) when the capsule 106 is detached from the holding portion 103.

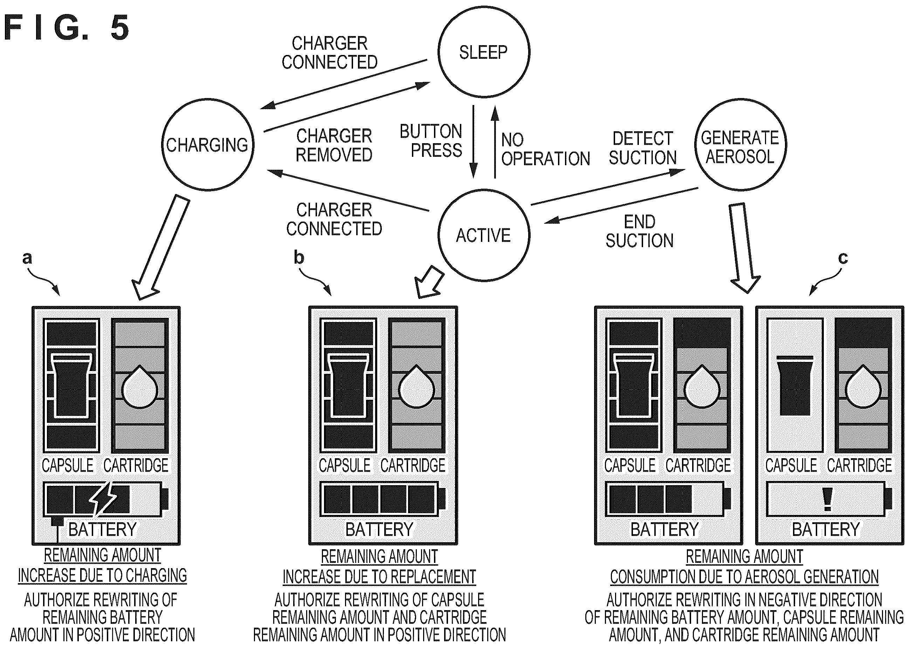

The state transition of the power supply unit 102 or the inhalation apparatus 100 and a display example of the second display D2 in each state according to the embodiment will be described with reference to a state transition diagram shown in Fig. 5. The power supply unit 102 can have four operation modes of a sleep mode, an active mode, an aerosol generation mode, and a charging mode. Note that the first display D1 may also be controlled to perform display, similar to the second display D2. Alternatively, the first display D1 and the third display D3 may be controlled to display simplified display contents of the second display D2. Alternatively, the first display D1 and the third display D3 may display information for complementing information displayed on the second display D1.

The sleep mode is a state in which the power supply unit 102 stops the main operation. In the sleep mode, no electric power for heating the aerosol source is supplied to the heater 127 of the atomizer or cartridge 104. In the sleep mode, the electric power consumed by the power supply unit 102 can be minimized. The sleep mode can also be called a power-saving mode or a standby mode. In the sleep mode, the aerosol providing function of the power supply unit 102 is locked, and thus the user cannot inhale aerosol.

In the sleep mode, if a predetermined operation is performed on the action button B, the lock is released, and the power supply unit 102 shifts to the active mode. The predetermined operation can be, for example, an operation of repeatedly pressing the action button B a predetermined number of times (for example, three times), an operation of long-pressing the action button B for a predetermined time (for example, 3 sec), or the like. If, in the active mode, a predetermined time elapses without the predetermined operation, the power supply unit 102 or the inhalation apparatus 100 can return to the sleep mode.

If the puff sensor 209 detects suction (a puff) by the user in the active mode, the power supply unit 102 shifts to the aerosol generation mode of generating aerosol. When the suction ends or a suction time reaches a predetermined upper limit time, the power supply unit 102 can return to the active mode. The active mode and the aerosol generation mode may be understood as the first mode in which it is possible to control discharge from the power supply 205 to the heater 127, and the sleep mode may be understood as the second mode in which the power consumption is smaller than in the first mode. In accordance with the operation of the action button B, the power supply unit 102 may shift from the active mode to the aerosol generation mode. In this case, the power supply unit 102 can return to the active mode when suction is detected a predetermined number of times or a predetermined time elapses in the aerosol generation mode.

When the external power supply (charger) is connected to the V_{bus} port in the sleep mode or the active mode, the inhalation apparatus 100 shifts to the charging mode and the power supply 205 is charged. When the external power supply is detached from the V_{bus} port or the power supply is set in a full charge state, the inhalation apparatus 100 shifts to the sleep mode. The full charge state means that the SOC (State Of Charge) or the charging rate is 100% or is equal to or higher than a predetermined value (for example, 98%, 95%, or 90%) close to 100%. The power supply unit 102 or the inhalation apparatus 100 may have a function of causing the user to set a criterion on which the full charge state is determined.

In Fig. 5, b shows a display example of the second display D2 in the active mode. In this display example, the second display D2 displays, in a bar graph form, each of the remaining amount (to be referred to as the "capsule remaining amount" hereinafter) of the flavor source 131 in the capsule 106, the remaining amount (to be referred to as the "cartridge remaining amount" hereinafter) of the aerosol source in the cartridge 104, and the remaining amount of electric power dischargeable by the power supply 205 (to be referred to as the "remaining battery amount" hereinafter). In Fig. 5, a shows a display example of the second display D2 in the charging mode. In the charging mode as well, the capsule remaining amount, the cartridge remaining amount, and the remaining battery amount can be displayed. In an area where the remaining battery amount is displayed, a charging mark representing a charging state can additionally be displayed. In Fig. 5, c shows display examples of the second display D2 in the aerosol generation mode. Since the capsule remaining amount, the cartridge remaining amount, and the remaining battery amount respectively decrease by generating aerosol, the number of bars in at least one bar graph decreases, as shown in a left portion in c of Fig. 5. A right portion in c of Fig. 5 shows that the capsule remaining amount is small and there is no remaining battery amount. In this way, the second display D2 can be controlled by the processor 207 to include display concerning an element consumed to generate flavored aerosol, for example, at least one, at least two, or all of the capsule remaining amount, the cartridge remaining amount, and the remaining battery amount.

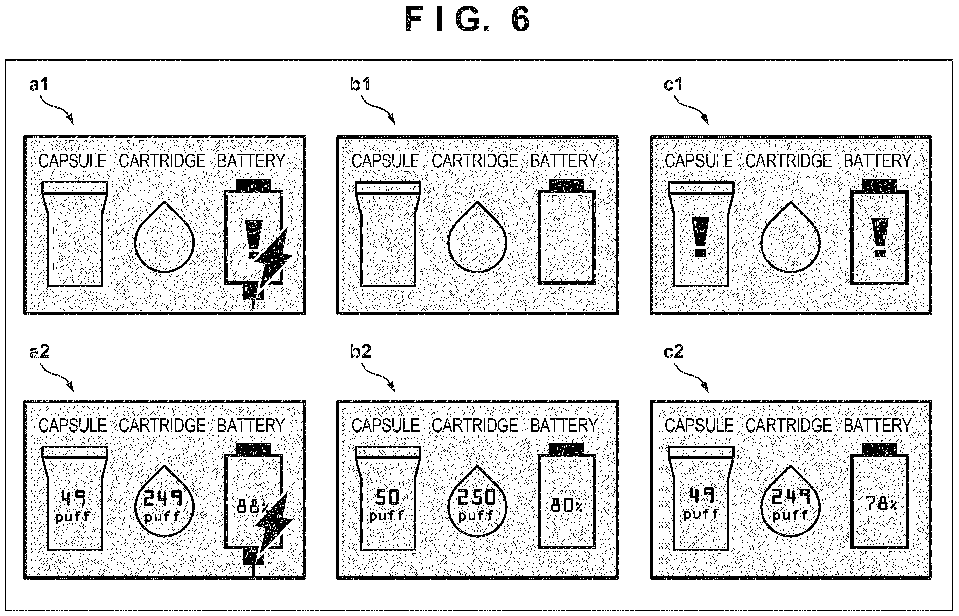

The display forms shown in Fig. 5 are merely examples, and the display forms of the capsule remaining amount, the cartridge remaining amount, and the remaining battery amount may be other forms. For example, as shown in a1, b1, and c1 of Fig. 6, icons representing only the presence/absence of the remaining amounts of the plurality of elements, respectively, may be displayed. As exemplified in a1, b1, and c1 of Fig. 6, the display form of the second display D2 can include display for specifying one of two states with respect to at least one of the plurality of elements. The two states can include a first state indicating that the capability to generate flavored aerosol is sufficient and a second state indicating that the capability to generate flavored aerosol is insufficient. In the examples in a1, b1, and c1 of Fig. 6, an icon without a mark "!" represents the first state, and an icon with the mark "!" represents the second state. In Fig. 6, a1 shows that the capsule remaining amount and the cartridge remaining amount are in the first state and the remaining battery amount is in the second state. In Fig. 6, b1 shows that the capsule remaining amount, the cartridge remaining amount, and the remaining battery amount are in the first state. In Fig. 6, c1 shows that the cartridge remaining amount is in the first state and the capsule remaining amount and the remaining battery amount are in the second state. As exemplified in b1, b2, and b3 of Fig. 6, the display form of the second display D2 may be such that each remaining amount is indicated by a numerical value.

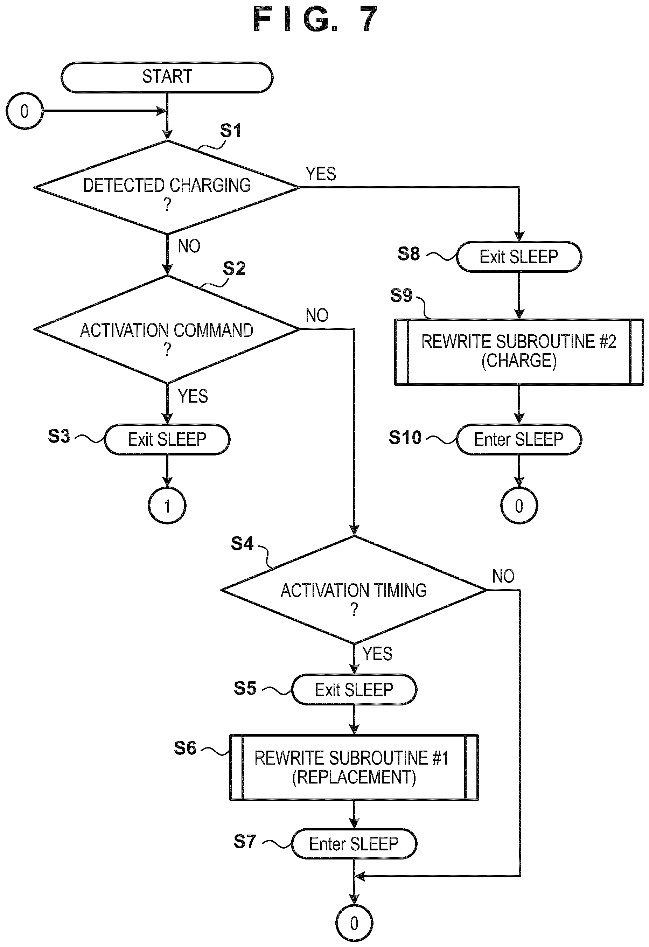

An example of the operation of the power supply unit 102 or the inhalation apparatus 100 will be described with reference to Figs. 7 to 12. This operation is controlled by the processor 207. In the initial state, the power supply unit 102 stands by in the sleep mode. In step S1, the processor 207 determines whether the external power supply (charger) is connected to the V_{bus} port to start charging of the power supply 205. If it is determined that charging is started, the process advances to step S8; otherwise, the process advances to step S2. In step S2, the processor 207 determines whether an activation command is generated by operating the action button B. If it is determined that the activation command is generated, the process advances to step S3; otherwise, the process advances to step S4. For example, if a predetermined operation of, for example, repeatedly pressing the action button B a predetermined number of times is performed, the processor 207 can determine that the activation command is generated. In step S3, the processor 207 leaves the sleep mode to shift to the active mode.

In step S4, the processor 207 determines whether a scheduled activation timing is reached. If it is determined that the scheduled activation timing is reached, the process advances to step S5; otherwise, the process returns to step S1. The scheduled activation timing can be, for example, a predetermined periodic timing (for example, every hour, every two hours, every three hours, or every 24 hours) or a preprogrammed timing (for example, 12:00 A.M.)

In step S5, the processor 207 leaves the sleep mode to shift to the active mode. Next, in step S6, the processor 207 executes a rewrite subroutine #1 as processing concerning display rewrite at the time of replacement of the cartridge or the capsule, then shifts from the active mode to the sleep mode in step S7, and returns the process to step S1.

If charging is detected in step S1, the processor 207 leaves the sleep mode to shift to the charging mode in step S8, and executes a rewrite subroutine #2 as processing concerning display rewrite at the time of charging. After that, the processor 207 shifts from the charging mode to the sleep mode in step S10, and returns the process to step S1. Details of the rewrite subroutine #1 and the rewrite subroutine #2 will be described later.

As described above, the processor 207 shifts from the sleep mode to the active mode in accordance with the activation command or the scheduled activation timing, and executes processing (rewrite subroutine #1) concerning display rewrite at the time of replacement of the cartridge or the capsule. Furthermore, the processor 207 shifts from the sleep mode to the charging mode at the start of charging, and executes processing (rewrite subroutine #2) concerning display rewrite at the time of charging.

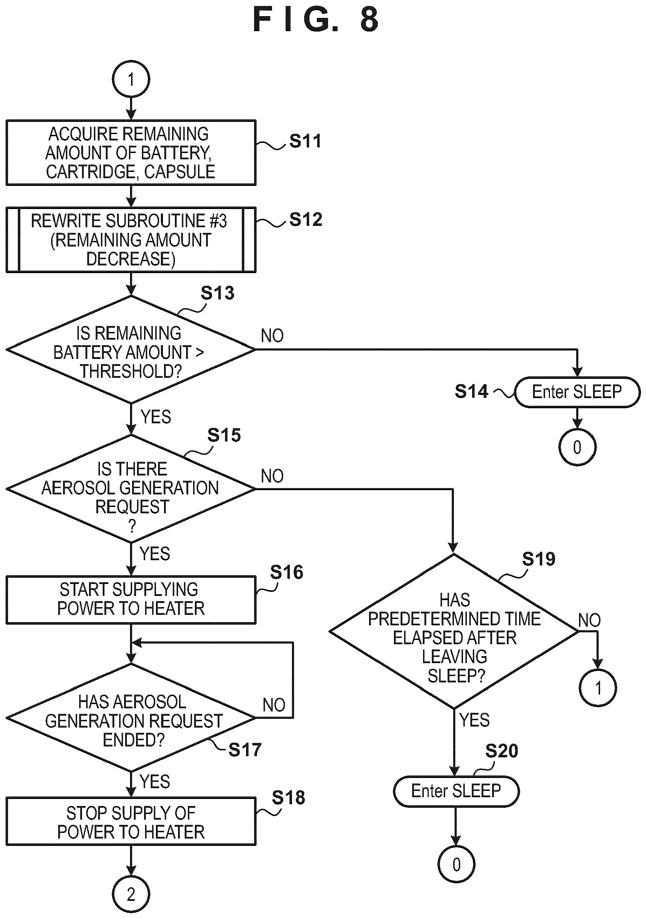

Fig. 8 shows a control procedure after the processor 207 leaves the sleep mode to shift to the active mode in step S3. Note that although not shown, after shifting to the active mode, the rewrite subroutine #1 can be executed repeatedly (for example, periodically) at the scheduled timing along with the following processing, and the rewrite subroutine #2 can also be executed when charging is started. When charging is started, the control procedure shown in Fig. 8 can forcibly be ended.

From step S11, the remaining amount of each of the plurality of elements that can be displayed using the display D2 (or at least one of the first display D1 and the third display D3 in addition to the display D2, and this also applies below) decreases except for a case where cartridge replacement, capsule replacement, or charging is performed. To cope with this, in step S11, the processor 207 acquires the remaining amounts of the plurality of elements, that is, the remaining battery amount, the cartridge remaining amount, and the capsule remaining amount. After that, in step S12, the processor 207 executes a rewrite subroutine #3 as processing concerning rewrite of the display D2 along with a decrease in the remaining amount of each element.

In step S13, the processor 207 determines whether the remaining battery amount is larger than a threshold. The threshold is, for example, a threshold for determining whether the remaining battery amount permits the operation in the active mode and the aerosol generation mode. More specifically, the threshold can be set as, for example, the predetermined lower limit value of the remaining battery amount with which even generation of aerosol corresponding to N (for example, N = 1) puff operations is impossible. If the remaining battery amount is equal to or smaller than the threshold, the processor 207 determines that it cannot operate in the active mode or the aerosol generation mode, and shifts to the sleep mode in step S14, and the process returns to step S1.

If the remaining battery amount is larger than the threshold, the processor 207 waits, in step S15, for generation of an aerosol generation request (atomization request). The aerosol generation request can be, for example, transmission or a notification of detection of puff from the puff sensor 209 to the processor 207. Alternatively, if an operation unit such as a switch or a sensor (neither is shown) for an aerosol generation request is provided, an aerosol generation request can be generated by operating the operation unit by the user. An aerosol generation request may be generated by operating the action button B.

If the generation of the aerosol generation request is detected in step S15, the processor 207 starts electric power supply to the heater 127 (the processor 207 shifts to the aerosol generation mode) in step S16. After that, in step S17, the processor 207 waits for the end of the aerosol generation request. If the aerosol generation request ends, the processor 207 stops the electric power supply to the heater 127 (the processor 207 shifts to the active mode) in step S18.

If no aerosol generation request is generated in step S15, the processor 207 determines, in step S19, whether a predetermined time has elapsed after leaving the sleep mode. If the predetermined time has elapsed after leaving the sleep mode, the processor 207 returns to the sleep mode in step S20, and the process returns to step S1. If the predetermined time has not elapsed after leaving the sleep mode, the processor 207 returns the process to step S11. Instead, the processor 207 may return the process to step S15.

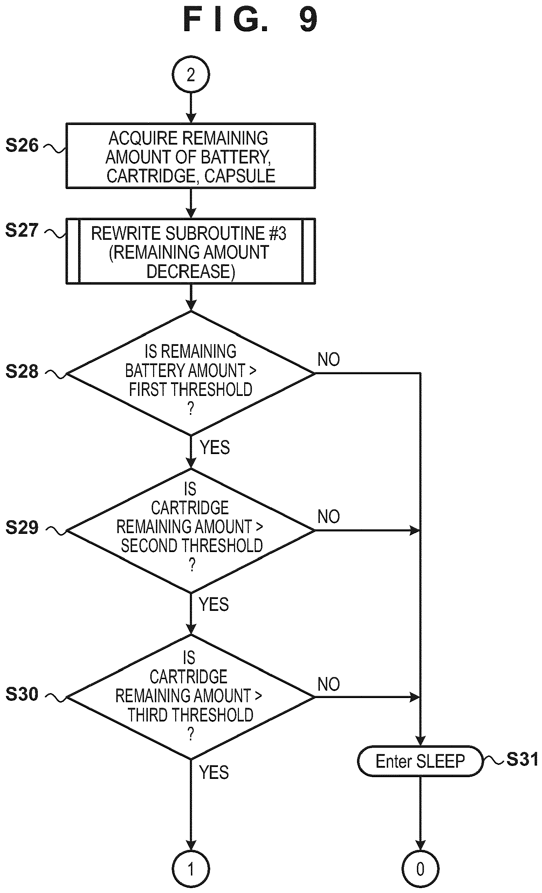

Fig. 9 shows a control procedure after the electric power supply to the heater 127 is stopped in step S18. In step S26, the processor 207 acquires the remaining amounts of the plurality of elements, that is, the remaining battery amount, the cartridge remaining amount, and the capsule remaining amount. After that, in step S27, the processor 207 executes the rewrite subroutine #3 as processing concerning rewrite of the display D2 along with a decrease in the remaining amount of each element. The processor 207 can acquire the remaining battery amount by, for example, acquiring the output voltage of the power supply 205 or the number of times of puff after the completion of charging. Alternatively, if the power supply unit 102 includes a management circuit that manages the power supply 205, the processor 207 can acquire the remaining battery amount based on an output from the management circuit. The processor 207 can acquire the cartridge remaining amount based on, for example, the number of times of puff after the cartridge 104 is attached to the power supply unit 102 or the holding portion 103 or based on, if a sensor that detects the remaining amount is provided, an output from the sensor. The processor 207 can acquire the capsule remaining amount based on, for example, the number of times of puff after the capsule 106 is attached to the power supply unit 102 or the holding portion 103 or based on, if a sensor that detects the remaining amount is provided, an output from the sensor.

The cartridge 104 may have a function of providing identification information such as RF-ID. In this case, the processor 207 can manage the cartridge remaining amount of each cartridge 104 based on the identification information of the cartridge 104. Similarly, the capsule 106 may have a function of providing identification information such as RF-ID. In this case, the processor 207 can manage the capsule remaining amount of each capsule 106 based on the identification information of the capsule 106.

In step S28, the processor 207 determines whether the remaining battery amount is larger than a first threshold. If it is determined that the remaining battery amount is larger than the first threshold, the process advances to step S29. In step S29, the processor 207 determines whether the cartridge remaining amount is larger than a second threshold. If it is determined that the cartridge remaining amount is larger than the second threshold, the process advances to step S30. In step S30, the processor 207 determines whether the capsule remaining amount is larger than a third threshold. If it is determined that the capsule remaining amount is larger than the third threshold, that is, if each of the remaining amounts of the plurality of elements is larger than the corresponding threshold, the process returns to step S11. In this case, the process may return not to step S11 but to step S15.

If it is determined in step S28 that the remaining battery amount is equal to or smaller than the first threshold, if it is determined in step S29 that the cartridge remaining amount is equal to or smaller than the second threshold, or if it is determined in step S30 that the capsule remaining amount is equal to or smaller than the third threshold, the processor 207 shifts to the sleep mode in step S31, and the process returns to step S1. If it is determined in step S28 that the remaining battery amount is equal to or smaller than the first threshold, the processor 207 can notify, using at least one of the displays D 1, D2, and D3 and the vibration generation unit V, the user of a warning indicating that charging should be performed, before shifting to the sleep mode. If it is determined in step S29 that the cartridge remaining amount is equal to or smaller than the second threshold, the processor 207 can notify, using at least one of the displays D1, D2, and D3 and the vibration generation unit V, the user of a warning indicating that the cartridge 104 should be replaced, before shifting to the sleep mode. If it is determined in step S30 that the capsule remaining amount is equal to or smaller than the third threshold, the processor 207 can notify, using at least one of the displays D 1, D2, and D3 and the vibration generation unit V, the user of a warning indicating that the capsule 106 should be replaced, before shifting to the sleep mode.

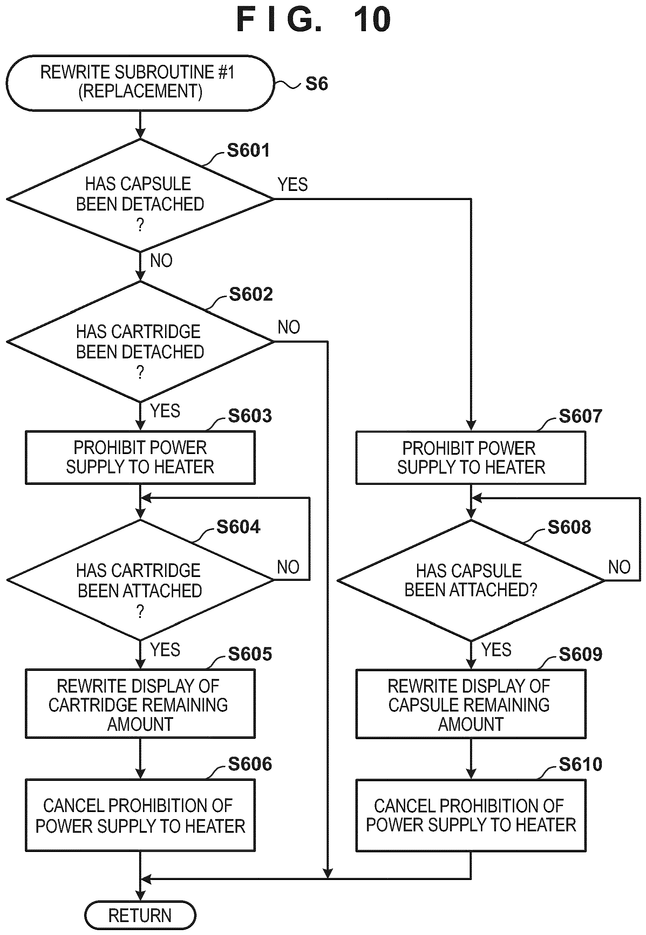

Fig. 10 shows a control procedure of the rewrite subroutine #1 executed in step S6. In step S601, the processor 207 determines whether the capsule 106 is detached from the power supply unit 102 or the holding portion 103. If it is determined that the capsule 106 is detached, the process advances to step S607; otherwise, the process advances to step S602. If, for example, the sensor 222 is provided, the processor 207 can determine detachment of the capsule 106 from the power supply unit 102 or the holding portion 103 based on an output from the sensor 222. Alternatively, if detachment and attachment of the capsule 106 appear as a change in output from the measurement circuit 210, the processor 207 can determine detachment of the capsule 106 from the power supply unit 102 or the holding portion 103 based on the change in output from the measurement circuit 210.

In step S602, the processor 207 determines whether the cartridge 104 is detached from the power supply unit 102 or the holding portion 103. If it is determined that the cartridge 104 is detached, the process advances to step S603; otherwise, the rewrite subroutine #1 is ended. If, for example, the sensor 221 is provided, the processor 207 can determine detachment of the cartridge 104 from the power supply unit 102 or the holding portion 103 based on an output from the sensor 221. Alternatively, the processor 207 can determine detachment of the cartridge 104 from the power supply unit 102 or the holding portion 103 based on a change in output from the measurement circuit 210. In this example, in a state in which the cartridge 104 is correctly held by the holding portion 103, when the switch 201 is turned on, a voltage obtained by dividing the output voltage of the voltage converter 202 by the heater 127 and the shunt resistor Rₛₕᵤₙₜ is supplied to the measurement circuit 210. On the other hand, in a state in which the cartridge 104 is detached from the power supply unit 102 or the holding portion 103, when the switch 201 is turned on, the output voltage of the voltage converter 202 is supplied to the measurement circuit 210. Therefore, if the cartridge 104 is detached from the power supply unit 102 or the holding portion 103, when the switch 201 is turned on, a signal supplied from the measurement circuit 210 to the processor 207 changes.

In step S603, the processor 207 prohibits electric power supply to the heater 127. In a state in which electric power supply to the heater 127 is prohibited, even if an aerosol generation request is generated, no electric power is supplied to the heater 127.

In step S604, the processor 207 waits for attachment of the cartridge 104 to the power supply unit 102 or the holding portion 103. If, for example, the sensor 221 is provided, the processor 207 can determine attachment of the cartridge 104 to the power supply unit 102 or the holding portion 103 based on an output from the sensor 221. Alternatively, the processor 207 can determine attachment of the cartridge 104 to the power supply unit 102 or the holding portion 103 based on a change in output from the measurement circuit 210.

In step S605, the processor 207 rewrites the display of the cartridge remaining amount (the remaining amount of the aerosol source of the cartridge 104) to a remaining amount corresponding to replacement of the cartridge 104. Typically, the processor 207 rewrites the display of the second display D2 so that the new cartridge remaining amount is larger than the original cartridge remaining amount. In an example, the processor 207 can rewrite the display of the second display D2 so that the cartridge remaining amount is displayed as 100%. The power supply unit 102 may include a remaining amount sensor that detects the remaining amount of the aerosol source of the cartridge 104. In this case, the processor 207 may rewrite the display of the second display D2 so as to display a cartridge remaining amount corresponding to an output from the remaining amount sensor.

In step S606, the processor 207 cancels the prohibition of the electric power supply to the heater 127, and ends the rewrite subroutine #1. Thus, when an aerosol generation request is generated, a state in which it is possible to supply electric power to the heater 127 is set accordingly.

If it is determined in step S601 that the capsule 106 is detached from the power supply unit 102 or the holding portion 103, the processor 207 prohibits, in step S607, electric power supply to the heater 127. In a state in which electric power supply to the heater 127 is prohibited, even if an aerosol generation request is generated, no electric power is supplied to the heater 127.

In step S608, the processor 207 waits for attachment of the capsule 106 to the power supply unit 102 or the holding portion 103. If, for example, the sensor 222 is provided, the processor 207 can determine attachment of the capsule 106 to the power supply unit 102 or the holding portion 103 based on an output from the sensor 222. Alternatively, if detachment and attachment of the capsule 106 appear as a change in output from the measurement circuit 210, the processor 207 can determine attachment of the capsule 106 to the power supply unit 102 or the holding portion 103 based on the change in output from the measurement circuit 210.

In step S609, the processor 207 rewrites the display of the capsule remaining amount (the remaining amount of the flavor material of the capsule 106) to a remaining amount corresponding to replacement of the capsule 106. Typically, the processor 207 rewrites the display of the second display D2 so that the new capsule remaining amount is larger than the original capsule remaining amount. In an example, the processor 207 can rewrite the display of the second display D2 so that the capsule remaining amount is displayed as 100%. The power supply unit 102 may include a remaining amount sensor that detects the remaining amount of the flavor material of the capsule 106. In this case, the processor 207 may rewrite the display of the second display D2 so as to display a capsule remaining amount corresponding to an output from the remaining amount sensor.

In step S610, the processor 207 cancels the prohibition of the electric power supply to the heater 127, and ends the rewrite subroutine #1. Thus, when an aerosol generation request is generated, a state in which it is possible to supply electric power to the heater 127 is set accordingly.

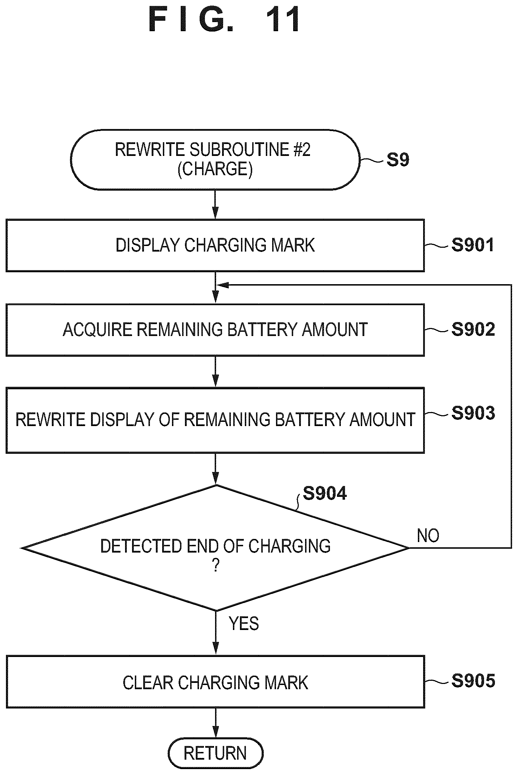

Fig. 11 shows a control procedure of the rewrite subroutine #2 executed in step S9. In step S901, the processor 207 rewrites the display of the second display D2 so as to display a charging mark representing that the power supply 205 is currently charged. In step S902, the processor 207 acquires the remaining battery amount (the remaining amount of electric power dischargeable by the power supply 205). The processor 207 can acquire the remaining battery amount, for example, by acquiring the output voltage of the power supply 205, based on the number of times of puff after the completion of charging, or, if a management circuit that manages the power supply 205 is provided, based on an output from the management circuit.

In step S903, the processor 207 rewrites the display of the second display D2 so as to display the remaining battery amount acquired in step S902. As exemplified in Fig. 5, the remaining battery amount can be display in a bar graph form. In step S904, the processor 207 determines, based on the remaining battery amount acquired in step S902, whether charging of the power supply 205 ends. If charging ends, the process advances to step S905; otherwise, the process returns to step S902. In step S905, the processor 207 rewrites the display of the second display D2 so as to clear the display of the charging mark, and ends the rewrite subroutine #2.

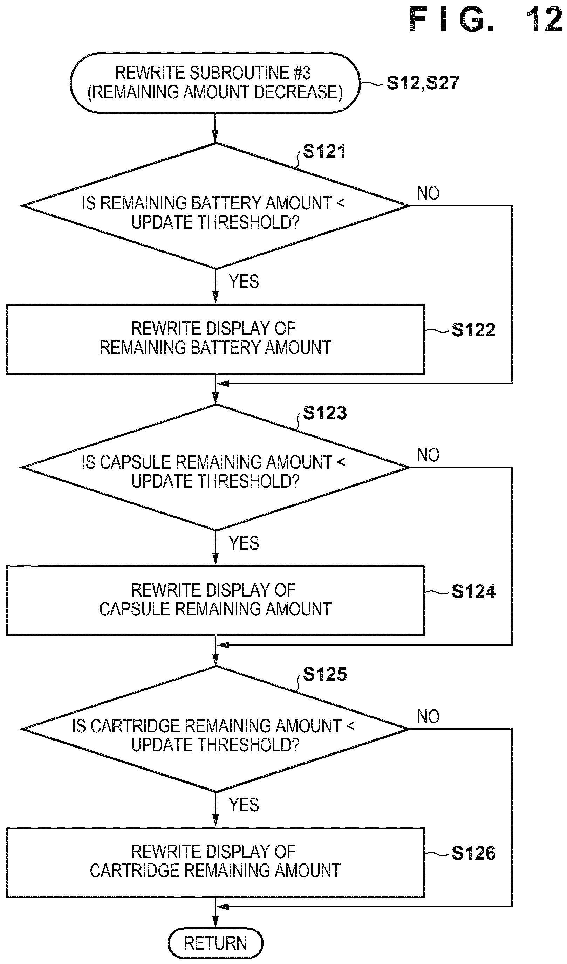

Fig. 12 shows a control procedure of the rewrite subroutine #3 executed in steps S12 and S27. In step S121, the processor 207 determines whether the remaining battery amount is smaller than a first update threshold. If the remaining battery amount is smaller than the first update threshold, the process advances to step S122; otherwise, the process advances to step S123 (the process skips step S 122). In step S122, the processor 207 rewrites the display of the second display (electronic paper display) D2 so as to display a remaining battery amount corresponding to the current remaining battery amount (or the current first update threshold) acquired immediately before the rewrite subroutine #3 is executed. As exemplified in Fig. 5, the remaining battery amount can be displayed in a bar graph form. In step S122, the processor 207 may change the first update threshold from the current value to a smaller value. Thus, the remaining battery amount can be displayed in a plurality of levels, as exemplified as the bar graph display in Fig. 5.

In step S 123, the processor 207 determines whether the capsule remaining amount is smaller than a second update threshold. If the capsule remaining amount is smaller than the second update threshold, the process advances to step S124; otherwise, the process advances to step S125 (the process skips step S124). In step S124, the processor 207 rewrites the display of the second display D2 so as to display a capsule remaining amount corresponding to the current capsule remaining amount (or the current second update threshold) acquired immediately before the rewrite subroutine #3 is executed. As exemplified in Fig. 5, the capsule remaining amount can be displayed in a bar graph form. In step S124, the processor 207 may change the second update threshold from the current value to a smaller value. Thus, the capsule remaining amount can be displayed in a plurality of levels, as exemplified as the bar graph display in Fig. 5.

In step S 125, the processor 207 determines whether the cartridge remaining amount is smaller than a third update threshold. If the cartridge remaining amount is smaller than the third update threshold, the process advances to step S126; otherwise, the rewrite subroutine #3 is ended (the process skips step S126). In step S126, the processor 207 rewrites the display of the second display D2 so as to display a cartridge remaining amount corresponding to the current cartridge remaining amount (or the current third update threshold) acquired immediately before the rewrite subroutine #3 is executed. As exemplified in Fig. 5, the cartridge remaining amount can be displayed in a bar graph form. In step S126, the processor 207 may change the third update threshold from the current value to a smaller value. Thus, the cartridge remaining amount can be displayed in a plurality of levels, as exemplified as the bar graph display in Fig. 5.

Note that in a case where the remaining battery amount is displayed in a bar graph form, as exemplified in Fig. 5, the first update threshold can include thresholds the number of which is equal to the number of bars. In step S121, the processor 207 can compare the remaining battery amount with the threshold corresponding to the current remaining battery amount among the thresholds included in the first update threshold. This applies to a case where the capsule remaining amount or the cartridge remaining amount is displayed in a bar graph form. If each of the remaining battery amount after the battery is fully charged, the capsule remaining amount after replacement, and the cartridge remaining amount after replacement is a sufficient amount and the bar graph display is sufficiently discrete (the number of bars of the bar graph display is sufficiently small), it will be understood that the frequency of updating the display of the second display D2 by the processor 207 in accordance with the occurrence of the factor for changing the remaining amount of the generation source of aerosol is lower than the frequency of discharge from the power supply 205 to the heater 127. More specifically, each of the remaining battery amount after the battery is fully charged, the capsule remaining amount after replacement, and the cartridge remaining amount after replacement need only be an amount with which electric power supply to the heater 127 that is performed in response to an aerosol generation request can be performed the number of times that is larger than the number of bars of the bar graph display.

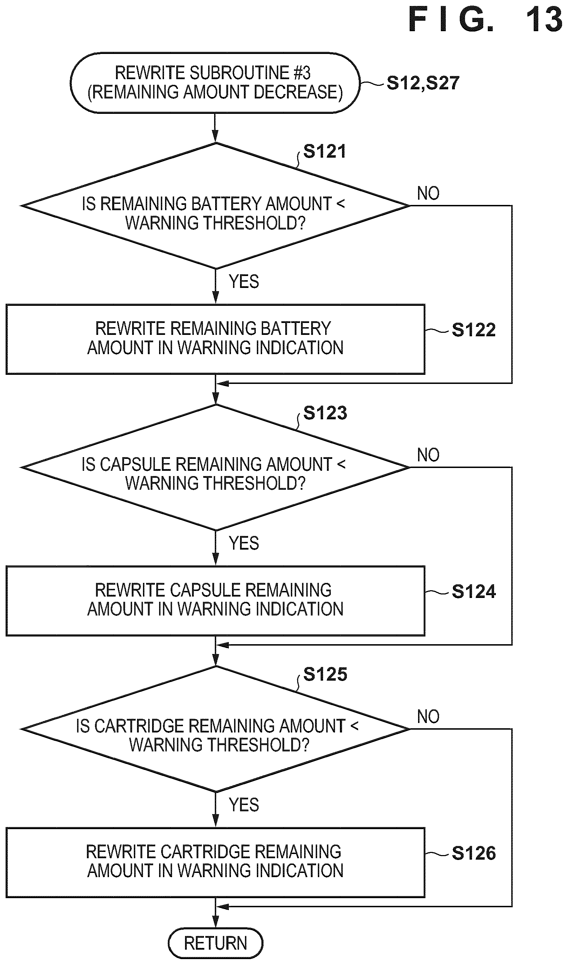

Fig. 13 shows a control procedure of a modification of the rewrite subroutine #3 executed in steps S12 and S27. In step S121, the processor 207 determines whether the remaining battery amount is smaller than a first warning threshold. If the remaining battery amount is smaller than the first warning threshold, the process advances to step S122; otherwise, the process advances to step S123 (the process skips step S122). In step S122, the processor 207 rewrites the display of the second display (electronic paper display) D2 so as to display a warning indicating that the remaining battery amount is insufficient or a warning for urging charging of the power supply 205.

In step S123, the processor 207 determines whether the capsule remaining amount is smaller than a second warning threshold. If the capsule remaining amount is smaller than the second warning threshold, the process advances to step S124; otherwise, the process advances to step S125 (the process skips step S124). In step S124, the processor 207 rewrites the display of the second display D2 so as to display a warning indicating that the capsule remaining amount is insufficient or that the replacement time of the capsule approaches.

In step S125, the processor 207 determines whether the cartridge remaining amount is smaller than a third warning threshold. If the cartridge remaining amount is smaller than the third warning threshold, the process advances to step S126; otherwise, the rewrite subroutine #3 is ended (the process skips step S126). In step S126, the processor 207 rewrites the display of the second display D2 so as to display a warning indicating that the cartridge remaining amount is insufficient or that the replacement time of the cartridge approaches.

As described above, in the example shown in Figs. 7 to 12, the power supply unit 102 includes the action button B as an operation unit, and the processor 207 shifts from the sleep mode to the active mode in step S3 when the action button B is operated in step S2, and then updates the generation source remaining amount display (the display of the cartridge remaining amount and the capsule remaining amount). Furthermore, the processor 207 shifts from the sleep mode to the active mode at the scheduled timing in step S5, confirms replacement of the generation sources (the cartridge and the capsule) in step S6, and updates the generation source remaining amount display when the generation sources are replaced.

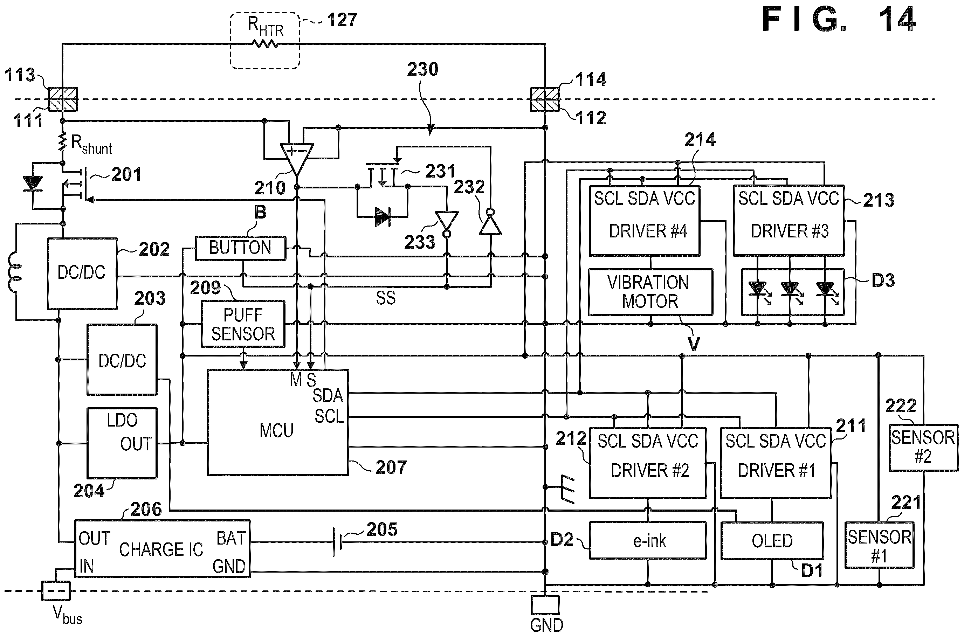

Fig. 14 shows an example of the arrangement of the electrical component 110 having the function of detecting replacement of the cartridge 104 in the sleep mode. The output of the action button B is connected to an input terminal S of the processor 207 via an activation signal line SS. The processor 207 can recognize a user operation on the action button B based on a signal supplied from the action button B. If the user presses the action button B, the action button B outputs a signal of an active level (a high-active signal in this example of the arrangement), and this signal is supplied to the input terminal S of the processor 207. If a predetermined operation is performed on the action button B in the sleep mode, the processor 207 (power supply unit 102) shifts to the active mode.

The predetermined operation can be, for example, an operation of repeatedly pressing the action button B a predetermined number of times (for example, three times) or an operation of pressing the action button B for a predetermined time (for example, 3 sec) or more. For example, if the operation of repeatedly pressing the action button B the predetermined number of times (for example, three times) is performed, a signal of high level can be supplied to the input terminal S the predetermined number of times. Alternatively, if the operation of pressing the action button B for the predetermined time (for example, 3 sec) or more is performed, a signal of high level can be supplied to the input terminal S for the predetermined time (for example, 3 sec) or more. If the predetermined operation is performed on the action button B in the sleep mode, the power supply unit 102 can shift from the sleep mode to the active mode.

The power supply unit 102 can include a detection circuit 230 that detects replacement of the cartridge 104 in the sleep mode. The detection circuit 230 can be configured to cause the signal level of the activation signal line SS to transition to high level as an active level when, for example, the cartridge 104 is replaced or attached. The detection circuit 230 can include, for example, a transistor (PMOSFET) 231, a first inverter 232, and a second inverter 233. The input terminal of the first inverter 232 can be connected to the activation signal line SS, and the output terminal of the first inverter 232 can be connected to the gate of the transistor 231. The input terminal of the second inverter 233 can be connected to the source of the transistor 231, and the output terminal of the second inverter 233 can be connected to the activation signal line SS. The drain of the transistor 231 can be connected to the output of the measurement circuit 210.

In a state in which the cartridge 104 is correctly attached to the power supply unit 102 or the holding portion 103, the measurement circuit 210 supplies a voltage corresponding to the resistance value R_{HTR} of the heater 127 to an input terminal M of the processor 207. The measurement circuit 210 is configured so that this voltage becomes a voltage falling within a predetermined range. In a state in which the cartridge 104 is detached from the power supply unit 102 or the holding portion 103, the measurement circuit 210 supplies a voltage higher than the upper limit value of the predetermined range to the input terminal M of the processor 207. More specifically, in the state in which the cartridge 104 is correctly attached to the power supply unit 102 or the holding portion 103, the measurement circuit 210 supplies, to the input terminal M of the processor 207, a voltage (in other words, a voltage corresponding to the resistance value R_{HTR} of the heater 127) corresponding to a voltage obtained by dividing the output voltage of the voltage converter 202 by the heater 127 and the shunt resistor Rₛₕᵤₙₜ. In the state in which the cartridge 104 is detached from the power supply unit 102 or the holding portion 103, the measurement circuit 210 supplies a voltage corresponding to the output voltage of the voltage converter 202 to the input terminal M of the processor 207. It is apparent that the output voltage of the voltage converter 202 is higher than the voltage obtained by dividing the output voltage of the voltage converter 202 by the heater 127 and the shunt resistor Rₛₕᵤₙₜ.

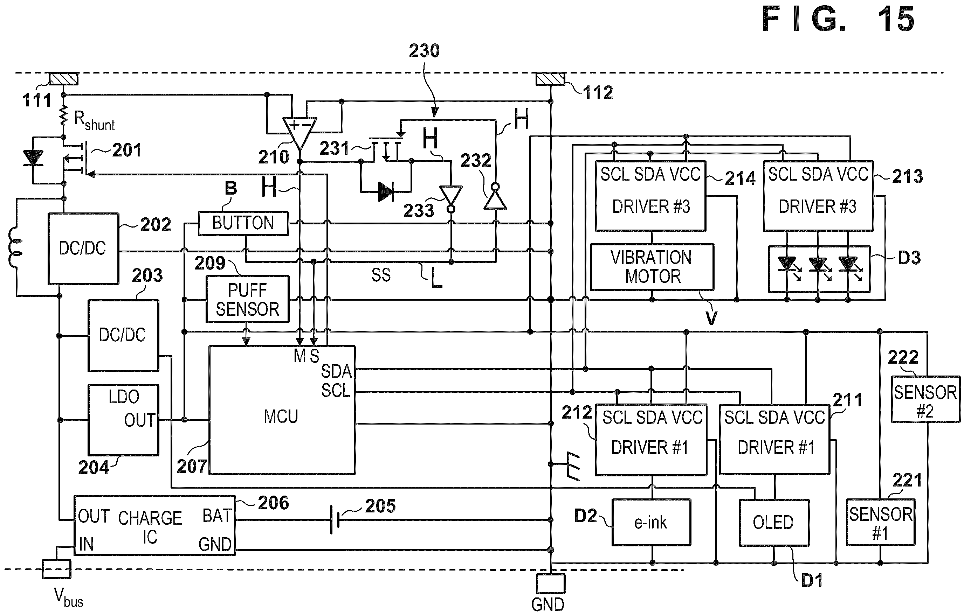

Fig. 15 shows the state in which the cartridge 104 is detached from the power supply unit 102 or the holding portion 103 in the example of the arrangement shown in Fig. 14. In Fig. 15, L and H represent low level and high level, respectively. In the state in which the cartridge 104 is detached, low level (L) as an inactive level is supplied to the input terminal S of the processor 207. More specifically, high level supplied from the measurement circuit 210 is supplied to the second inverter 233 via the body diode of the transistor 231, and the second inverter 233 supplies, to the input terminal S of the processor 207 via the activation signal line SS, low level obtained by logically inverting high level. On the other hand, high level as a voltage indicating detachment of the cartridge 104 from the power supply unit 102 or the holding portion 103 is supplied to the input terminal M of the processor 207. Therefore, the processor 207 can detect detachment of the cartridge from the power supply unit 102 or the holding portion 103 based on the voltage supplied to the input terminal S and the input terminal M.

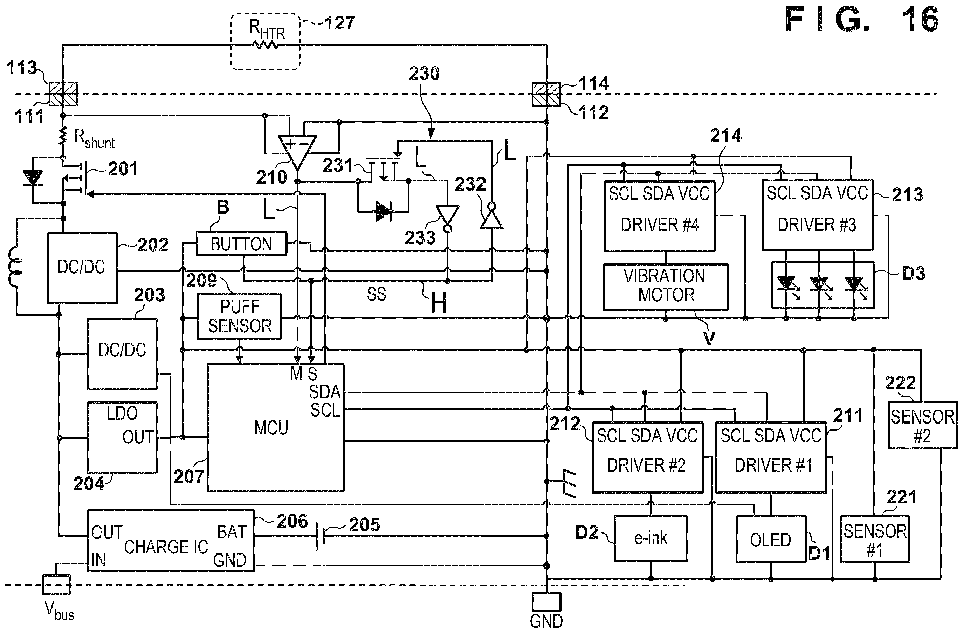

Fig. 16 shows the state in which the cartridge 104 is attached to the power supply unit 102 or the holding portion 103 (that is, the state in which the cartridge 104 is replaced) in the example of the arrangement shown in Fig. 14. In Fig. 16, L and H represent low level and high level, respectively. In the state in which the cartridge 104 is attached, high level (H) as an active level is input to the input terminal S of the processor 207. More specifically, low level (that is not a ground level) supplied from the measurement circuit 210 is supplied to the second inverter 233 via the body diode of the transistor 231, and the second inverter 233 supplies, to the input terminal S of the processor 207 via the activation signal line SS, high level obtained by logically inverting low level. Therefore, the processor 207 can detect that the cartridge 104 is replaced when the signal supplied to the input terminal S shifts from low level to high level in the state in which the cartridge 104 is detached. As described above, the electrical signal obtained from the current path (the path formed from the voltage converter 202, the switch 201, the shunt resistor Rₛₕᵤₙₜ, and the heater 127) formed by holding the cartridge 104 by the holding portion 103 and the output signal of the action button B are supplied to the input terminal S of the processor 207. The processor 207 can detect the command of shifting from the second mode to the first mode and replacement of the cartridge 104 based on the signals supplied to the input terminal S. After the processor 207 recognizes or detects replacement of the cartridge 104, the voltage of the activation signal line SS can be reset to low level by a reset circuit (not shown) that can be controlled by the processor 207.

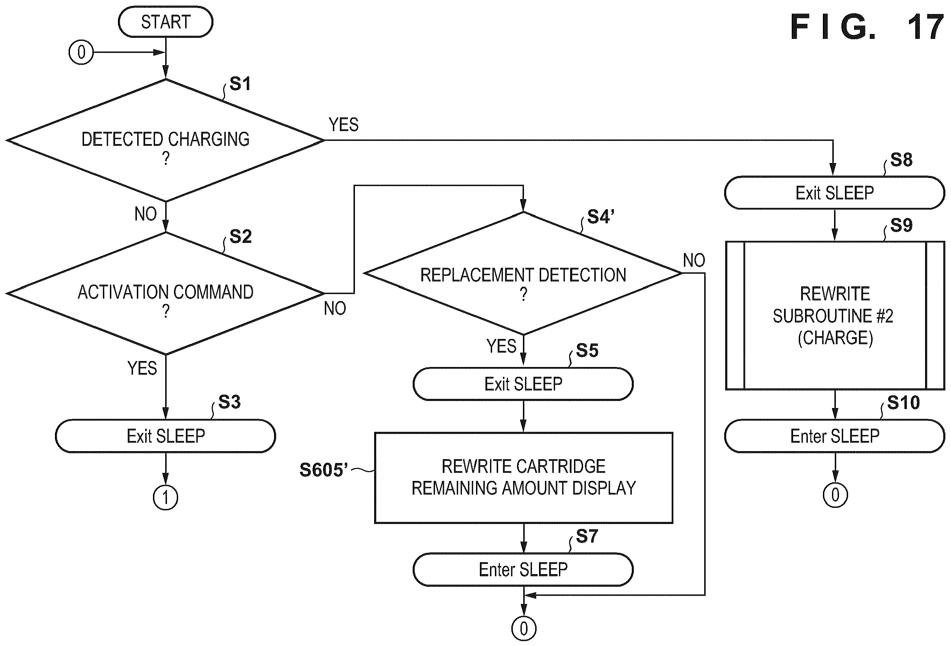

Fig. 17 shows a control procedure in the example of the arrangement shown in Fig. 14. The control procedure shown in Fig. 17 is obtained by replacing steps S4 and S605 in the control procedure shown in Fig. 7 by steps S4' and S605', respectively. Step S4' is executed in the sleep mode. In step S4', the processor 207 can detect replacement of the cartridge 104 when the signal supplied to the input terminal S shifts from low level to high level in the state in which the cartridge 104 is detached, as described above. If replacement of the cartridge 104 is detected in step S4', the processor 207 leaves the sleep mode to shift to the active mode in step S5, executes, in step S605', the same processing as that in step S605 described above, and then shifts to the sleep mode in step S7.

More specifically, in step S605', the processor 207 can rewrite the display of the second display (electronic paper display) D2 so that the cartridge remaining amount (the remaining amount of the aerosol source of the cartridge 104) in the display (generation source remaining amount display) is larger than the remaining amount in the original display. In an example, the processor 207 can rewrite the display of the second display D2 so that the cartridge remaining amount is displayed as 100%. The power supply unit 102 may include a remaining amount sensor that detects the remaining amount of the aerosol source of the cartridge 104. In this case, the processor 207 may rewrite the display of the second display D2 so as to display a cartridge remaining amount corresponding to an output from the remaining amount sensor.

As described above, in the example of the arrangement shown in Figs. 14 to 17, the display of the second display D2 includes the generation source remaining amount display concerning the remaining amount of the cartridge 104 as the generation source of aerosol. Furthermore, in a case where the cartridge 104 is replaced in the sleep mode (second mode) in which the power consumption is smaller than in the active mode (first mode), the processor 207 can update the generation source remaining amount display. In a case where the cartridge 104 is replaced in the second mode, the processor 207 can update the generation source remaining amount display after shifting to the active mode. After that, the processor 207 can shift to the sleep mode. The processor 207 can detect, as replacement of the cartridge 104, that the cartridge 104 is detached from the holding portion 103 and then a new cartridge 104 is attached to the holding portion 103.

In the example of the arrangement described with reference to Figs. 14 to 17, replacement of the capsule 106 is not detected. However, for example, replacement of the capsule 106 can be detected based on an output from the sensor 222. In this case, the capsule remaining amount can be rewritten, similar to the rewrite of the display of the cartridge remaining amount in accordance with replacement of the cartridge, which has been described with reference to Fig. 17.

Furthermore, replacement of the cartridge 104 and replacement of the capsule 106 are detected based on outputs from the sensors 221 and 222, and thus the display of the cartridge remaining amount and the capsule remaining amount may be updated.

The invention is not limited to the foregoing embodiments, and various variations/changes are possible within the spirit of the invention.

### REFERENCE SIGNS LIST

100: inhalation apparatus, 102: controller for inhalation apparatus, 103: holding portion, 104: atomizer, 125: container, 126: transport portion, 127: heater, D1, D2, D3: display, 207: processor

## Claims

1. A controller for an inhalation apparatus, wherein the controller operates by electric power supplied from a power supply, and comprises:
a holding portion configured to hold an atomizer including a heater configured to generate flavored aerosol from a generation source of an aerosol source;
a nonvolatile display; and
a processor configured to control update of display of the nonvolatile display,
wherein the processor determines whether to perform the update in response to occurrence of a factor for changing a remaining amount of the generation source.

2. The controller for the inhalation apparatus, according to claim 1, wherein
the factor includes discharge from the power supply to the heater, and
the processor performs, after an end of the discharge, the update of the display of the nonvolatile display while the discharge is not performed.

3. The controller for the inhalation apparatus, according to claim 2, wherein the processor does not perform the update of the display of the nonvolatile display while the discharge is performed.

4. The controller for the inhalation apparatus, according to claim 2 or 3, wherein the display of the nonvolatile display includes display concerning a remaining amount of the power supply.

5. The controller for the inhalation apparatus, according to any one of claims 1 to 4, wherein the factor includes replacement of the generation source.

6. The controller for the inhalation apparatus, according to claim 5, wherein the display of the nonvolatile display includes display concerning the remaining amount of the generation source.

7. The controller for the inhalation apparatus, according to claim 5, wherein
the generation source includes a first generation source as a generation source of aerosol, and a second generation source as a generation source of a flavor,
the factor includes replacement of the first generation source, and
the factor includes replacement of the second generation source.

8. The controller for the inhalation apparatus, according to claim 7, wherein the display of the nonvolatile display includes display concerning a remaining amount of the first generation source and display concerning a remaining amount of the second generation source.

9. The controller for the inhalation apparatus, according to any one of claims 1 to 8, wherein the display of the nonvolatile display includes display concerning a remaining amount of at least one element consumed to generate the flavored aerosol.

10. The controller for the inhalation apparatus, according to claim 9, wherein the display of the nonvolatile display includes bar graph display.

11. The controller for the inhalation apparatus, according to claim 9, wherein the display of the nonvolatile display includes display for specifying one of two states with respect to at least one of the at least one element.

12. The controller for the inhalation apparatus, according to claim 11, wherein the two states include a first state indicating that a capability of generating the flavored aerosol is sufficient and a second state indicating that the capability of generating the flavored aerosol is insufficient.

13. The controller for the inhalation apparatus, according to any one of claims 1 to 3, wherein
the processor includes a first mode in which discharge from the power supply to the heater can be controlled and a second mode in which power consumption is smaller than in the first mode,
the display of the nonvolatile display includes generation source remaining amount display concerning the remaining amount of the generation source, and
in a case where the generation source is replaced in the second mode, the processor updates the generation source remaining amount display.

14. The controller for the inhalation apparatus, according to claim 13, wherein in a case where the generation source is replaced in the second mode, the processor updates the generation source remaining amount display after shifting to the first mode.

15. The controller for the inhalation apparatus, according to claim 13 or 14, wherein the processor detects, as replacement of the generation source, that the generation source is detached from the holding portion and then a new generation source is attached to the holding portion.

16. The controller for the inhalation apparatus, according to claim 15, wherein the processor detects replacement of the generation source based on an electrical signal obtained from a current path formed by holding the generation source by the holding portion.

17. The controller for the inhalation apparatus, according to claim 16, further comprising an operation unit,
wherein the processor shifts from the second mode to the first mode in response to an operation of the operation unit, and
the processor includes an input terminal configured to be supplied with the electrical signal obtained from the current path and a signal corresponding to an output signal of the operation unit, and detects a command of shifting from the second mode to the first mode and replacement of the generation source based on the signals supplied to the input terminal.

18. The controller for the inhalation apparatus, according to claim 15, further comprising a sensor configured to detect presence or absence of the generation source,
wherein the processor detects replacement of the generation source based on an output from the sensor.

19. The controller for the inhalation apparatus, according to claim 13, further comprising an operation unit,
wherein the processor shifts from the second mode to the first mode in response to an operation of the operation unit, and
in a case where the generation source is replaced in the second mode, the processor shifts to the first mode in response to an operation of the operation unit, and then updates the generation source remaining amount display.

20. The controller for the inhalation apparatus, according to any one of claims 1 to 3, wherein
the processor includes a first mode in which discharge from the power supply to the heater can be controlled and a second mode in which power consumption is smaller than in the first mode,
the display of the nonvolatile display includes generation source remaining amount display concerning the remaining amount of the generation source, and
the processor shifts from the second mode to the first mode at a scheduled timing to confirm replacement of the generation source, and updates, in a case where the generation source is replaced, the generation source remaining amount display.

21. The controller for the inhalation apparatus, according to any one of claims 1 to 19, wherein a frequency of performing the update by the processor is lower than a frequency of discharge from the power supply to the heater.
